# EUROPEAN PATENT SPECIFICATION

(11) **EP 3 614 945 B1**
(45) Date of publication and mention of the grant of the patent: **12.06.2024**
(21) Application number: 18732213.6
(22) Date of filing: 29.05.2018
(51) Int. Cl.: A61B 18/14, A61B 5/00, A61M 25/00

(54) **ELECTROPHYSIOLOGY DEVICE WITH ELECTRODES HAVING INCREASED SURFACE AREA**
ELEKTROPHYSIOLOGIEVORRICHTUNG MIT ELEKTRODEN MIT ERHÖHTER OBERFLÄCHENGRÖSSE
DISPOSITIF D'ÉLECTROPHYSIOLOGIE À ÉLECTRODES AYANT UNE AIRE DE SURFACE ACCRUE

(30) Priority: 08.06.2017 US 201762516740 P
(43) Date of publication of application: 04.03.2020
(73) Proprietor: St. Jude Medical, Cardiology Division, Inc., St. Paul, MN 55117 (US)
(72) Inventor: OLSON, Gregory K., Elk River Minnesota 55330 (US); ORNBERG, Andreas, West St. Paul Minnesota 55118 (US); PARSONAGE, Edward E., St. Paul Minnesota 55116 (US); SPENCER, Chelsea, Minneapolis Minnesota 55406 (US)
(74) Representative: Mathys & Squire
(86) International application number: PCT/US2018/034922
(87) International publication number: WO 2018/226455

(56) References cited:
- EP-A1- 1 151 726
- WO-A1-2008/115665
- WO-A1-2018/053278
- WO-A2-2015/061250
- ARNE KLOKE ET AL: "Strategies for the Fabrication of Porous Platinum Electrodes", ADVANCED MATERIALS, vol. 23, no. 43, 16 November 2011 (2011-11-16), pages 4976-5008, XP055082182, ISSN: 0935-9648, DOI: 10.1002/adma.201102182

## Description

### BACKGROUND

The instant disclosure relates to catheters for use in medical procedures, such as electrophysiology studies. In particular, the instant disclosure relates to electrophysiology catheters that include electrodes with increased surface area and decreased impedance, both achieved by forming a porous surface on the electrodes.

Catheters are used for an ever-growing number of procedures, such as diagnostic, therapeutic, and ablative procedures, to name just a few examples. Typically, the catheter is manipulated through the patient's vasculature and to the intended site, for example, a site within the patient's heart.

A typical electrophysiology catheter as known from WO2008/115665 A1 includes an elongate shaft and one or more electrodes on the distal end of the shaft. The electrodes may be used for ablation, diagnosis, or the like. Oftentimes, these electrodes include ring electrodes that extend about the entire circumference of the catheter shaft, as well as a tip electrode.

As the dimensions of a measurement electrode decrease, the complex AC impedance with respect to a counter-electrode will generally increase. The increased impedance associated with smaller measurement electrodes can have undesirable effects during electrophysiology studies, such as electroanatomical mapping.

There are two primary contributing factors to the increased impedance. A first contributing factor is related to the dimensional dependence of the volumetric resistance (*i.e.,* smaller electrodes have regions of higher current density). A second contributing factor is related to the capacitance of the electrode (*i.e.,* as electrode dimensions shrink, the ionic AC current can become limited by how much charge can build up at the electrode surface). The second contributing factor, therefore, depends upon the total microscopic surface area of the electrode versus the macroscopic dimensional surface area of the electrode.

Various surface treatments are known to increase the surface area of a measurement electrode. Extant surface treatments, however, typically add material (*e.g*., iridium oxide; titanium nitride) to the measurement electrode, which complicates the manufacturing process. For example, porous electrodes are known from WO2015/061250 A2 and EP 1151 726 A.

### BRIEF SUMMARY

Disclosed herein is a method, according to the invention, of manufacturing an electrophysiology catheter, including:
forming a catheter body; forming a least one porous ablation electrode according to a process including: forming a substrate of a first more noble metal; forming an alloy including a second more noble metal and a less noble metal which is less noble than the first noble metal and the second more noble metal on the substrate; and de-alloying the alloy to form a porous matrix consisting essentially of the second more noble metal; and securing the at least one porous ablation electrode to the catheter body. The first more noble metal and the second more noble metal are selected from the group consisting of gold, platinum, and copper; the first more noble metal differs from the second more noble metal. The less noble metal is selected from the group consisting of silver, zinc, and lead.

According to aspects of the disclosure, the alloy can be formed by co-depositing the second more noble metal and the less noble metal on the substrate via electrochemical plating. In other aspects of the disclosure, the alloy can be formed by co-depositing the second more noble metal and the less noble metal on the substrate via electroless plating. In still other aspects of the disclosure, the alloy can be formed by co-depositing the second more noble metal and the less noble metal on the substrate via physical vapor deposition. In further aspects of the disclosure, the alloy can be formed by co-depositing the second more noble metal and the less noble metal on the substrate via chemical vapor deposition. In yet further aspects of the disclosure, the alloy can be formed by: applying a layer of the less noble metal to a layer of the second more noble metal; and heating the layer of the less noble metal and the layer of the second more noble metal to allow inter-diffusion of the less noble metal and the second more noble metal.

De-alloying the alloy can include electrochemically dissolving the less noble metal from the alloy

Also disclosed herein is an electrophysiology catheter, according to the invention, formed according to a process including: forming a catheter body; forming a least one porous electrode according to a process including: forming a substrate of a first more noble metal; forming an alloy including a second more noble metal and a less noble metal on the substrate; and de-alloying the alloy to form a porous matrix consisting essentially of the second more noble metal; and securing the at least one porous electrode to the catheter body. The first more noble metal and the second more noble metal are selected from the group consisting of gold, platinum, and copper, and are different from each other. The less noble metal is selected from the group consisting of silver, zinc, and lead.

The alloy can be formed by co-depositing the second more noble metal and the less noble metal on the substrate. For example, the second more noble metal and the less noble metal can be co-deposited via electrochemical plating, via electroless plating, via physical vapor deposition, and/or by chemical vapor deposition.

Alternatively, the alloy can be formed by: applying a layer of the less noble metal to a layer of the second more noble metal; and heating the layer of the less noble metal and the layer of the second more noble metal to allow inter-diffusion of the less noble metal and the second more noble metal.

The alloy can be de-alloyed by electrochemically dissolving the less noble metal from the alloy.

The instant disclosure also provides an electrophysiology catheter including: a body; and at least one electrode disposed on the body, wherein the at least one electrode includes a substrate consisting essentially of a first noble metal and a porous matrix consisting essentially of a second noble metal on the substrate.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 schematically depicts an electrophysiology catheter and associated systems.
Figure 2 is a close-up view of the distal region of the catheter shown in Figure 1.
Figure 3A schematically illustrates a porous electrode prior to a de-alloying process.
Figure 3B schematically illustrates a porous electrode after a de-alloying process.
Figure 4 is a microscopic photograph of a porous electrode according to aspects of the instant disclosure.

### DETAILED DESCRIPTION

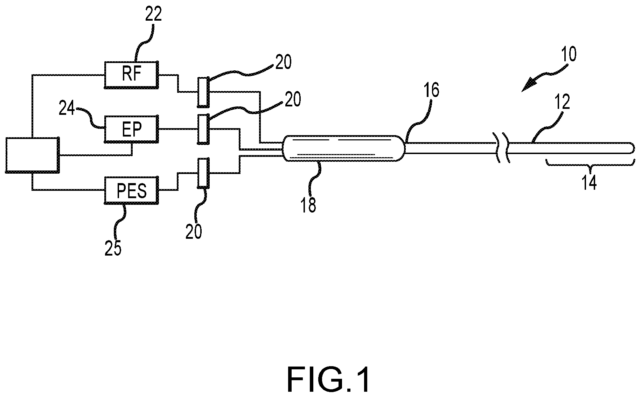

For purposes of illustration, the present teachings will be described in connection with a multi-electrode mapping and ablation catheter 10, such as illustrated in Figure 1. As shown in Figure 1, catheter 10 generally includes an elongate catheter body 12 having a distal region 14 and a proximal end 16. A handle 18 is shown coupled to proximal end 16. Figure 1 also shows connectors 20. Connectors 20 are configured to be connected to a source of ablation energy (schematically illustrated as RF source 22, which can be, for example, the Ampere^{™} RF ablation generator of Abbott Laboratories), an electrophysiology mapping device (schematically illustrated as 24, which can be, for example, the EnSite Precision^{™} cardiac mapping system, also of Abbott Laboratories), and a programmable electrical stimulator (schematically illustrated as 25, which can be, for example the EP-4^{™} cardiac stimulator, also of Abbott Laboratories). Although Figure 1 depicts three separate connectors 20, it is within the scope of the instant disclosure to have a combined connector 20 that is configured for connection to two or more of RF source 22, electrophysiology mapping device 24, and programmable electrical stimulator 25.

Various additional aspects of the construction of catheter 10 will be familiar to those of ordinary skill in the art. For example, the person of ordinary skill in the art will recognize that catheter 10 can be made steerable, for example by incorporating an actuator into handle 18 that is coupled to one or more steering wires that extend through elongate catheter body 12 and that terminate in one or more pull rings within distal region 14. Likewise, the ordinarily skilled artisan will appreciate that catheter 10 can be an irrigated catheter, such that it can also be coupled to a suitable supply of irrigation fluid and/or an irrigation pump. As a further example, those of ordinary skill in the art will appreciate that catheter 10 can be equipped with force feedback capabilities.

Insofar as such features are not necessary to an understanding of the instant disclosure, they are neither illustrated in the drawings nor explained in detail herein. By way of example only, however, catheter 10 can incorporate various aspects and features the following catheters, all from Abbott Laboratories: the EnSite^{™} Array^{™} catheter; the FlexAbility^{™} ablation catheter; the Safire^{™} BLU^{™} ablation catheter; the Therapy^{™} Cool Path^{™} irrigated ablation catheter; the Livewire^{™} TC ablation catheter; and the TactiCath^{™} Quartz irrigated ablation catheter.

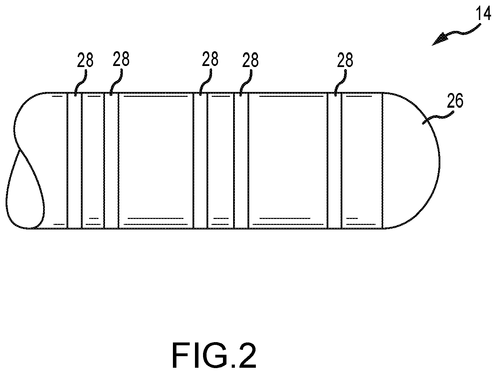

Figure 2 is a close-up of distal region 14 of catheter 10. Distal region 14 of catheter 10 includes a tip electrode 26 positioned at its distal end and a plurality of additional electrodes 28 proximal of tip electrode 26. In particular, Figure 2 depicts five ring electrodes 28. The person of ordinary skill in the art will understand and appreciate, however, that by varying the size (*e.g.,* width) and spacing of electrodes 28, different diagnostic and/or therapeutic objectives and/or outcomes can be achieved. For example, the ordinarily skilled artisan will appreciate that, as electrodes 28 become smaller and closer together, the electrograms collected thereby will become sharper and more localized evidencing better depiction of local, near-field depolarization of the cardiac tissue in contact with the electrodes. Thus, it should be understood that distal region 14 can include any number of such electrodes 28 (*e.g.,* 9 electrodes 28 for a decapolar catheter 10) and that the inter-electrode spacing can vary along the length of distal region 14.

Electrodes 28 may include any metal capable of detecting and conducting the local electrical signal. Suitable materials for electrodes 28 include, without limitation, platinum and gold.

Electrodes 28 can also be of various physical configurations. These include, by way of example only, ring electrodes, segmented ring electrodes, partial ring electrodes, flexible circuit electrodes, balloon electrodes, and spot electrodes. Various configurations of electrodes 28 (as well as electrode 26) are disclosed in International Publication No. WO 2016/182876.

The instant disclosure provides electrodes having a porous surface for increased microscopic surface area. More specifically, the instant disclosure provides noble metal electrodes including a matrix of pores that is formed through a de-alloying process (referred to herein as a "porous electrode"). Porous coatings formed by de-alloying processes will be familiar to those of ordinary skill in the art. *See, e.g.,* Erlebacher et al., Evolution of Nanoporosity in Dealloying, Nature 410, 450-453 (March 22, 2001).
Thus, de-alloying processes will be described herein only to the extent necessary to understand the instant disclosure.

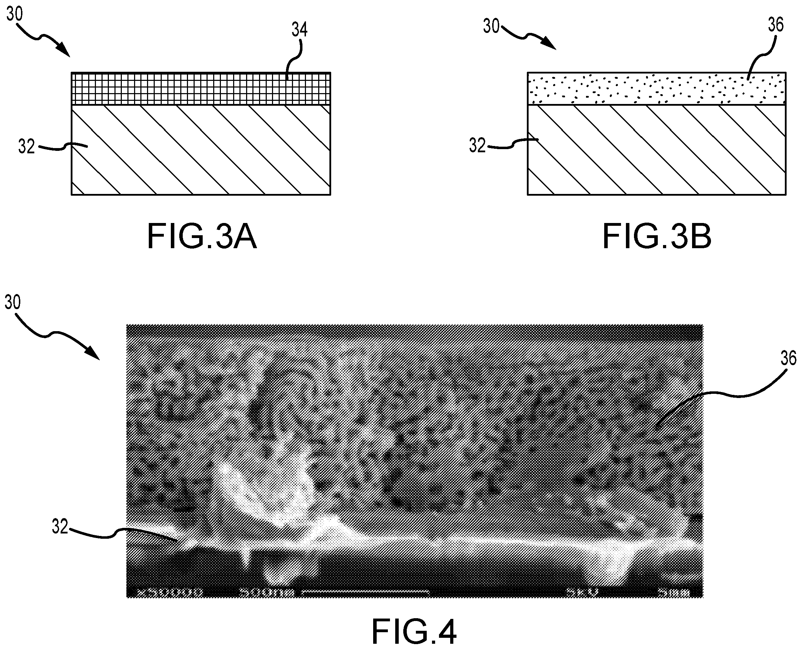

Figure 3A schematically illustrates a porous electrode 30 prior to a de-alloying process. As shown in Figure 3A, porous electrode 30 includes a substrate 32 and an alloy layer 34. Substrate 32 is made of a more noble metal, such as platinum, gold, or copper, while alloy layer 34 is an alloy of a more noble metal (which is different from the more noble metal of substrate 32) and a less noble metal, such as silver, zinc, or lead.

In aspects of the disclosure, alloy layer 34 is formed by co-depositing the more- and lessnoble metals, such as by electroless plating, electrochemical plating, physical vapor deposition, or chemical vapor deposition from a solution or atmosphere that contains the desired metal components. In other aspects of the disclosure, alloy layer 34 is formed by depositing a thin layer of the less noble metal on the surface of the more noble metal and then heating the resultant multi-layer structure to allow inter-diffusion of the metals.

In a de-alloying process, the less noble metal is removed from alloy layer 34, such as by electrochemical dissolution. For example, alloy layer 34 can be subjected to a voltage that will oxidize and solubilize the less noble metal, while leaving other alloy constituent metals (including the more noble metal) intact. This can be accomplished, for example, by using a 3-electrode configuration including alloy layer 34, a counter-electrode, and a reference electrode. It is also contemplated to add catalysts and/or other additives to the electrolyte solution to optimize the de-alloying process.

Figure 3B schematically illustrates a porous electrode 30 after a de-alloying process. As shown in Figure 3B, substrate 32 remains, and alloy layer 34 has been converted to a porous layer 36 including the more noble metal and a matrix of pores. The matrix of pores in porous layer 36 is visible in the microscopic photograph of Figure 4.

It should be understood that the relative thicknesses of substrate 32 and alloy layer 34 are not to scale in Figure 3A. Likewise, it should be understood that the relative thicknesses of substrate 32 and porous layer 36 are not to scale in Figure 3B.

The matrix of pores in porous layer 36 can result in an increase in the microscopic surface area of porous layer 36 by about 20 times to about 40 times relative to a solid metal electrode. Another advantage of porous layer 36 according to the instant disclosure is an
improvement in biosignal fidelity that results from a surface mechanical mismatch (as shown, for example, in connection with neuronal electrode development).

Although several embodiments have been described above with a certain degree of particularity, those skilled in the art could make numerous alterations to the disclosed embodiments without departing from the spirit or scope of this disclosure.

For example, the porous electrodes described herein can not only be formed prior to being attached to a catheter body, but can also be formed from non-porous electrodes already attached to a catheter body.

All directional references (*e.g*., upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g*., attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the invention as defined in the appended claims.

## Claims

1. A method of manufacturing an electrophysiology catheter (10), comprising:
forming a catheter body (12);
forming a least one porous ablation electrode (26, 28, 30) according to a process comprising:
forming a substrate (32) of a first more noble metal;
forming an alloy (34) comprising a second more noble metal and a less noble metal which is less noble than the first noble metal and the second more noble metal on the substrate, wherein the first more noble metal and the second more noble metal are selected from the group consisting of gold, platinum, and copper and wherein the less noble metal is selected from the group consisting of silver, zinc, and lead; and
de-alloying the alloy (34) to form a porous matrix consisting essentially of the second more noble metal; and
securing the at least one porous ablation electrode (26, 28, 30) to the catheter body (12),
wherein the first more noble metal differs from the second more noble metal.

2. The method according to claim 1, wherein forming the alloy (34) comprises co-depositing the second more noble metal and the less noble metal on the substrate (32) via electrochemical plating.

3. The method according to claim 1, wherein forming the alloy (34) comprises co-depositing the second more noble metal and the less noble metal on the substrate (32) via electroless plating.

4. The method according to claim 1, wherein forming the alloy (34) comprises co-depositing the second more noble metal and the less noble metal on the substrate (32) via physical vapor deposition.

5. The method according to claim 1, wherein forming the alloy (34) comprises co-depositing the second more noble metal and the less noble metal on the substrate (32) via chemical vapor deposition.

6. The method according to claim 1, wherein forming the alloy (34) comprises:
applying a layer of the less noble metal to a layer of the second more noble metal; and
heating the layer of the less noble metal and the layer of the second more noble metal to allow inter-diffusion of the less noble metal and the second more noble metal.

7. The method according to claim 1, wherein de-alloying the alloy (34) comprises electrochemically dissolving the less noble metal from the alloy (34).

8. An electrophysiology catheter (10) formed by a method according any one of claims 1 to 7.

## Patentansprüche

1. Verfahren zum Herstellen eines elektrophysiologischen Katheters (10), enthaltend:
Bilden eines Katheterkörpers (12);
Herstellen mindestens einer porösen Ablationselektrode (26, 28, 30) nach einem Vorgang, der enthält:
Herstellen eines Substrats (32) aus einem ersten edleren Metall;
Bilden einer Legierung (34), die ein zweites edleres Metall und ein weniger edles Metall enthält, das weniger edel ist als das erste edle Metall und das zweite edlere Metall, auf dem Substrat, wobei das erste edlere Metall und das zweite edlere Metall aus der Gruppe ausgewählt sind, die aus Gold, Platin und Kupfer besteht, und wobei das weniger edle Metall aus der Gruppe ausgewählt ist, die aus Silber, Zink und Blei besteht; und
Entlegieren der Legierung (34) zum Bilden einer porösen Matrix, die im Wesentlichen aus dem zweiten edleren Metall besteht; und
Befestigen der mindestens einen porösen Ablationselektrode (26, 28, 30) an dem Katheterkörper (12),
wobei sich das erste edlere Metall von dem zweiten edleren Metall unterscheidet.

2. Verfahren nach Anspruch 1, wobei das Bilden der Legierung (34) das gemeinsame Aufbringen des zweiten edleren Metalls und des weniger edlen Metalls auf das Substrat (32) durch elektrochemisches Überziehen enthält.

3. Verfahren nach Anspruch 1, wobei das Bilden der Legierung (34) das gemeinsame Aufbringen des zweiten edleren Metalls und des weniger edlen Metalls auf das Substrat (32) durch stromloses Überziehen enthält.

4. Verfahren nach Anspruch 1, wobei das Bilden der Legierung (34) das gemeinsame Aufbringen des zweiten edleren Metalls und des weniger edlen Metalls auf das Substrat (32) durch physikalische Dampf-Ablagerung enthält.

5. Verfahren nach Anspruch 1, wobei das Bilden der Legierung (34) das gemeinsame Aufbringen des zweiten edleren Metalls und des weniger edlen Metalls auf das Substrat (32) durch chemische Dampf-Ablagerung enthält.

6. Verfahren nach Anspruch 1, wobei das Bilden der Legierung (34) enthält:
Aufbringen einer Schicht des weniger edlen Metalls auf eine Schicht des zweiten edleren Metalls; und
Erhitzen der Schicht des weniger edlen Metalls und der Schicht des zweiten edleren Metalls zum Ermöglichen einer Interdiffusion des weniger edlen Metalls und des zweiten edleren Metalls.

7. Verfahren nach Anspruch 1, wobei das Entlegieren der Legierung (34) ein elektrochemisches Lösen des weniger edlen Metalls aus der Legierung (34) enthält.

8. Elektrophysiologie-Katheter (10), der durch ein Verfahren nach einem der Ansprüche 1 bis 7 hergestellt ist.

## Revendications

1. Procédé de fabrication d'un cathéter d'électrophysiologie (10), comprenant les étapes consistant à :
former un corps de cathéter (12) ;
former au moins une électrode d'ablation poreuse (26, 28, 30) selon un processus comprenant les étapes consistant à :
former un substrat (32) d'un premier métal plus noble ;
former un alliage (34) comprenant un deuxième métal plus noble et un métal moins noble qui est moins noble que le premier métal noble et le deuxième métal plus noble sur le substrat, dans lequel le premier métal plus noble et le deuxième métal plus noble sont choisis dans le groupe constitué de l'or, du platine et du cuivre et dans lequel le métal moins noble est choisi dans le groupe constitué de l'argent, du zinc et du plomb ; et
désallier l'alliage (34) pour former une matrice poreuse constituée essentiellement du deuxième métal plus noble ; et
fixer ladite au moins une électrode d'ablation poreuse (26, 28, 30) au corps de cathéter (12),
dans lequel le premier métal plus noble est différent du deuxième métal plus noble.

2. Procédé selon la revendication 1, dans lequel la formation de l'alliage (34) comprend le co-dépôt du deuxième métal plus noble et du métal moins noble sur le substrat (32) par placage électrochimique.

3. Procédé selon la revendication 1, dans lequel la formation de l'alliage (34) comprend le co-dépôt du deuxième métal plus noble et du métal moins noble sur le substrat (32) par placage sans électrolyse.

4. Procédé selon la revendication 1, dans lequel la formation de l'alliage (34) comprend le co-dépôt du deuxième métal plus noble et du métal moins noble sur le substrat (32) par dépôt physique en phase vapeur.

5. Procédé selon la revendication 1, dans lequel la formation de l'alliage (34) comprend le co-dépôt du deuxième métal plus noble et du métal moins noble sur le substrat (32) par dépôt chimique en phase vapeur.

6. Procédé selon la revendication 1, dans lequel la formation de l'alliage (34) comprend les étapes consistant à :
appliquer une couche du métal moins noble sur une couche du deuxième métal plus noble ; et
chauffer la couche du métal moins noble et la couche du deuxième métal plus noble pour permettre l'inter-diffusion du métal moins noble et du deuxième métal plus noble.

7. Procédé selon la revendication 1, dans lequel le désalliage de l'alliage (34) comprend la dissolution électrochimique du métal moins noble de l'alliage (34).

8. Cathéter d'électrophysiologie (10) formé par un procédé selon l'une quelconque des revendications 1 à 7.
